# EUROPÄISCHE PATENTSCHRIFT

(11) **EP 0 825 884 B1**
(45) Veröffentlichungstag und Bekanntmachung des Hinweises auf die Patenterteilung: **12.12.2001**
(21) Anmeldenummer: 96917382.2
(22) Anmeldetag: 17.05.1996
(51) Int. Cl.: A61M 5/28, B65D 75/34

(54) **Blisterpackung mit Injektionsvorrichtung**
Blister pack with injection device
Emballage du type blister avec dispositif d'injection

(30) Priorität: 19.05.1995 DE 19518426
(43) Veröffentlichungstag der Anmeldung: 04.03.1998
(73) Patentinhaber: SCHWARZ PHARMA AG, D-40789 Monheim/Rhld. (DE)
(72) Erfinder: KAHLERT, Ernst-Ulrich, D-40474 Düsseldorf (DE)
(86) Internationale Anmeldenummer: EP9602118
(87) Internationale Veröffentlichungsnummer: WO9636380

(56) Entgegenhaltungen:
- US-A- 2 618 263
- US-A- 3 736 933
- US-A- 5 019 048

## Beschreibung

Die vorliegende Erfindung betrifft eine Multiblockblisterpackung, bestehend aus mehreren Einheiten, die jeweils eine fertige Injektionsvorrichtung zum sofortigen Gebrauch darstellen.

Injektionsvorrichtungen, insbesondere Spritzen, finden in der täglichen Praxis vielfach Verwendung. Auf Grund ihrer Bedeutung zur Lösung unterschiedlicher Problemstellungen erstreckt sich ihr Gebrauch auf alle technischen und ethischen Bereiche des täglichen Lebens.

Ohne Anspruch auf Vollständigkeit finden sie beispielsweise zum Auffüllen von Hohlräumen mit unterschiedlichen Materialien zum punktgenauen oder linearen Verkleben von Einzelteilen oder Elementen, aber auch im Lebensmittelbereich z.B. im Back- und Konditoreigewerbe Anwendung.

In der ärztlichen Praxis sind Injektionsvorrichtungen zur Behandlung unterschiedlicher Krankheiten im Human- und Veterinärbereich ein unumgängliches, nicht wegzudenkendes Therapieinstrument.

Bei dieser Vielfalt der Einsatzmöglichkeiten der Injektionsvorrichtungen sind im Laufe der Zeit Variationen bzw. unterschiedliche Spritzentypen entstanden, um den speziellen Anforderungen ihres Einsatzes Rechnung zu tragen.

Insbesondere im medizinischen Bereich sind die verschiedensten Ausgestaltungen von Spritzen entwickelt worden.

Die Gestaltungsbreite reicht von der herkömmlichen wiederverwendbaren Spritze, deren Spritzenkörper aus Glas gefertigt ist und an den die Kanüle vor Gebrauch angesteckt wird, über technisch aufwendig gestaltete Injektoren bis zu einfachen Einmalspritzen, vorzugsweise solche aus Kunststoff, die mit und ohne Kanülen erhältlich sind. Solche Einmalspritzen aus Kunststoffmaterial sowie die mit einem genormten Adapter versehenen Metallkanülen werden in sogenannten Blisterpackungen zur Verfügung gestellt.

Bei der Einblisterung von Einmalspritzen und Kanülen ist die Kanüle häufig einsatzbereit auf den Konus der Einmalspritze aufgesteckt. Bei diesen Blisterbehältern können jedoch Schwierigkeiten dadurch entstehen, daß während des Transportes die Kanülen vom Spritzenkonus abfallen oder sich lockern. Im ersten Fall ist die Sterilität der Spritze wie auch der Kanüle bei der Entnahme aus dem Blister nicht mehr gewährleistet. Im zweiten Fall entstehen Schwierigkeiten dadurch, daß beim Aufziehen der Lösung, die injiziert werden soll, unsterile Luft angesaugt wird, oder daß die Flüssigkeit beim Injizieren zwischen Kanülenansatz und Spritzenkonus ausläuft.

Das Dokument DE-GBM 71 103 09.8 beschreibt eine als Aufreißpackung ausgeführte Blisterpackung, die mit einer Lasche am oberen Ende der Packung versehen ist, und den Spritzenkörper und die Kanüle, getrennt voneinander, enthält. Mittels der Lasche ist die Blisterpackung leicht zu öffnen und wird dabei nur so weit aufgerissen, daß der Spritzenkörper angehoben und in den Konus der ebenfalls nur wenig freigelegten Kanüle eingeschoben werden kann. Dies hat gegenüber anderen Konstruktionen von Blisterpackungen den Vorteil, daß der Spritzenkörper und die Kanüle unter dem Schutz der noch vorhandenen Blisterpackung unter aseptischen Bedingungen zusammengesetzt werden können.

Dabei ist jedoch weiterhin der Nachteil vorhanden, daß vor Gebrauch Spritzenkörper und Kanüle zusammengesetzt werden müssen und anschließend noch das zu injizierende Arzneimittel in die Spritze aufgezogen werden muß. Septische Probleme können bei diesen notwendigen Manipulationen nicht ausgeschlossen werden.

Aus der DE-AS 1 075 283 ist eine Spritzampulle zum einmaligen Gebrauch bekannt, bei der die Kanüle fest mit dem durch eine Membran verschlossenen Ampullenkörper verbunden ist. Vor dem Gebrauch wird diese Membran durch eine in einem Bolzen angebrachte Nadel durchstoßen, worauf eine aufgesetzte Verschlußhülse abgezogen wird und die Spritzampulle einsatzbereit ist. Jedoch ist bei dieser Konstruktion von Nachteil, daß die Fertigung der aus vielen Einzelhülsen bestehenden Einheit aufwendig ist und der Zusammenbau kompliziert ist. Weiterhin ist auch eine mängelfreie Handhabung nicht möglich. So läßt sich beispielsweise nicht vermeiden, daß nach dem Aufstechen der Verschlußmembran beim Abziehen der Verschlußhülse bereits Injektionslösung austreten kann, bevor die Spritze zum Einstich aufgesetzt wird.

Die DE 29 00 827 A1 beschreibt ebenfalls eine Spritzampulle zum einmaligen Gebrauch. Sie besteht aus einem leicht deformierbaren Ampullenkörper, einem Kanülenkörper mit fest eingepaßter Kanüle, aus dem die Kanüle beidseitig verschiebbar herausragt, und einer abziehbaren Schutzkappe. Nachteilig ist bei dieser Konstruktion, daß die Fertigung sehr aufwendig ist, und daß noch vor Benutzung der Spritzampulle diese zum Gebrauch durch Verschieben der Kanüle in den Ampullenkörper einsatzbereit gemacht werden muß. Ein weiterer Nachteil der Handhabung ist, daß die Schutzkappe vor Gebrauch abzuziehen ist. Schließlich ist nicht sichergestellt, daß die Schutzkappe der Kanüle beim Transport oder beim Manipulieren vor unmittelbarem Gebrauch abfällt, so daß nicht immer die erforderliche Sterilität gewährleistet ist.

Aus der US-A 3,989,045 ist eine mit einem Pharmakon vorgefüllte Spritze mit einer separaten Kanüle bekannt. Auch hier ist in einer Ausführungsform nachteilig, daß Ampulle und Kanüle getrennt sind und noch vor Gebrauch zusammengefügt werden müssen. Weiterhin ist dadurch nicht immer Sterilität gewährleistet.

In einer anderen Ausführungsform sind Spritzampulle und Kanüle fest miteinander verbunden und die Kanüle durch eine abziehbare Schutzkappe geschützt. Die Konstruktion dieser Schutzkappe gewährleistet jedoch keine Vorteile gegenüber den in den bereits zuvor genannten Dokumenten erwähnten Schutzkappenkonstruktionen.

Das Dokument US-A 4,130,117 beschreibt eine mit einem Medikament vorbefüllte Spritzampulle mit getrennter Kanüle. Zusätzlich enthält sie noch eine vor Gebrauch zu entfernende Membran. Die durch diese Konstruktionsmerkmale gekennzeichnete Spritzampulle weist weiterhin die zuvor genannten Nachteile auf und insbesondere die aufwendige Fertigung, d.h. das Zusammenfügen der Einzelteile zu einer gebrauchsfertigen Spritzampulle.

Die CH-PS 662 511 A5 offenbart ein Verfahren zur Herstellung einer zur Verwendung mit einer Subkutannadel bestimmten, mit Medikamenten gefüllten Ampulle. Nach diesem Verfahren werden Spritzampullen von der Art, wie sie in US-A 3,989,045 und US-A 4,130,117 beschrieben wurden, hergestellt. Die danach hergestellten Spritzampullen weisen daher die Nachteile auf, wie sie zuvor beschrieben wurden.

Zur Gewährleistung der Sterilität sollten daher Spritzen als Fertigspritzen, befüllt mit dem zu applizierenden Wirkstoff steril gefertigt, verpackt und als sogenannte Einmalspritzen oder Wegwerfspritzen ausgestaltet sein.

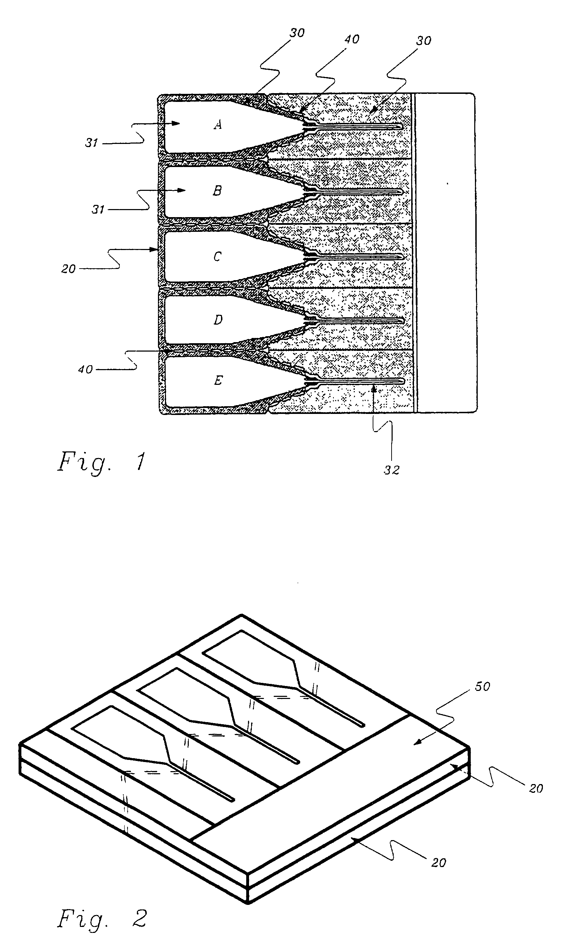

Das Dokument US-A 5,019,048 beschreibt eine Multiblockblisterpackung mit Injektionsvorrichtung zum sofortigen Gebrauch gemäß dem Oberbegriff des vorliegenden Anspruchs 1. Hierbei formen zwei kongruente, spiegelbildlich gegeneinander geschweißte Folien 13, 14 (Figs. 1, 2 und 7-10), die anliegende und nicht anliegende Bereiche aufweisen, mit ihren nicht anliegenden Bereichen zwei Taschen 56, 59. Tasche 56 macht dabei den mit Wirkstoff beladenen Spritzenkörper aus, mit dem eine Kanüle 55 ausklappbar verbunden ist. Die Kanüle 55 ragt in ein schlauchförmiges Ende der Tasche 56 hinein. Die Injektionsvorrichtung kann dabei als eine Einheit von mehreren in der Multiblockblisterpackung ausgebildet sein. Diese Multiblockblisterpackung ist als fortlaufendes Band, bestehend aus einer Vielzahl von Injektionsvorrichtungen, konstruiert. Bei dieser vorgeschlagenen Konstruktion einer Multiblockblisterpackung ist jedoch nachteilig, daß die einzelnen Injektionsvorrichtungen nur mit Hilfe einer speziellen Vorrichtung (Fig. 11) aus der Multiblockblisterpackung individualisiert werden können.

Injectionsvorrichtungen sollten jedoch problemlos und dennoch sicher zu handhaben sein und nicht nur dem behandelnden Arzt, sondern auch dem Patienten zur gefahrlosen Selbstmedikation zur Verfügung stehen sowie einfach und in der Fertigung kostengünstig gestaltet sein.

Aufgabe der vorliegenden Erfindung ist es, eine mit derartigen Vorteilen versehene Multiblockblisterpackung mit Injektionsvorrichtung zur Verfügung zu stellen.

Gelöst wird diese Aufgabe mit einer Multiblockblisterpackung gemäß Patentanspruch 1. Weiterbildungen sind in den Unteransprüchen beschrieben.

Die Injektionsvorrichtung ist dabei selbst als Grundeinheit Bestandteil eines Blistermultiblockes, wobei die spiegelbildlich gegeneinander geschweißten Folien der Multiblockblisterpackung und der Grundeinheit dieselben Folien sind.

Der Spritzenkörper der Injektionsvorrichtung wird durch die zwei kongruente Folien der Multiblockblisterpackung, die spiegelbildlich zueinander geschweißt sind und nicht aneinanderstoßende Teile aufweisen, die eine mit dem jeweiligen Wirkstoff beladene geformte Tasche bilden, dargestellt. Hieran schließt sich die Kanüle an, die mit einem Ende in die vorgenannte Tasche hineinragend, mit dieser eine untrennbare Einheit bildend, fest in Injektionsrichtung verbunden ist und deren restlicher Teil in eine weitere Tasche ragt, die ebenfalls von den zuvor genannten zwei kongruenten Folien, die spiegelbildlich zueinander geschweißt sind, gebildet wird.

Die Multiblockblisterpackung ist derart gestaltet, daß der Spritzenkörper aus durch Druck verformbarem Material besteht.

Innerhalb der Multiblockblistereinheit ist die Injektionsvorrichtung als eine Einheit von mindestens zwei miteinander verbundenen Einheiten einzeln abtrennbar. Vorzugsweise enthält eine Multiblockblistereinheit fünf bis zehn Einheiten der Injektionsvorrichtung.

Hierzu sind die Multiblockblistereinheiten mit geeigneten Perforationen versehen, die das Abtrennen einer gebrauchsfähigen Einheit vom Gesamtblock ermöglichen.

Die erfindungsgemäße Multiblockblisterpackung weist die folgende Ausführungsform auf.

Sie wird aus zwei kongruenten Folien (20), die spiegelbildlich zueinander geschweißt sind und sowohl aneinander anliegende Flächen (30) als auch nicht anliegende Hohlräume aufweisende Bereiche (31), die zu zwei Taschen geformt sind, gebildet. Die eine Tasche, [z.B. A (31)] macht den Spritzenkörper aus und ist mit dem jeweils zu injizierenden Wirkstoff beladen. Ihr zur Kanüle weisender Teil ist konisch zulaufend geformt, in den die Kanüle, mit der Tasche fest verbunden, mit einem Ende von definierter Länge, die die feste Verbindung sicherstellt, hineinragt. Diese Tasche weist umlaufend Schweißränder auf und besitzt an den von der Tasche wegweisenden Seiten Perforationen (40), soweit die Schweißränder innenliegend sind. Diese Perforationen bzw. Sollbruchstellen erlauben es, den Spritzenkörper mit der fest verbundenen Kanüle als fertige Injektionsvorrichtung zum sofortigen Gebrauch aus der Multiblockblisterpackung herauszutrennen.

Die andere, länglich geformte Tasche (32) nimmt die Kanüle auf. Beim Heraustrennen des Spritzenkörpers aus der Multiblockblisterpackung gleitet die mit dem Spritzenkörper fest verbundene Kanüle aus der Tasche heraus, die für die Kanüle eine schützende Verpackung darstellt.

Über die Gesamtlänge der Multiblockblisterpackung ist an der Seite zu der die Kanülen hinweisen, ein weiterer, durch eine Schweißnaht gebildeter Rand (50) angebracht, der verstärkt ausgebildet sein kann und das Heraustrennen der Injektionsvorrichtung, durch Zurverfügungstellung weiteren Haltes, erleichtern soll.

Die beiliegenden Zeichnungen erläutern die Erfindung näher.

Fig. 1 zeigt in der Draufsicht fünf nebeneinanderliegende Injektionsvorrichtungen in einer Multiblockblisterpackung.

Die Buchstaben A-E zeigen die als Taschen (31) gestalteten, mit Wirkstoff beladenen Spritzenkörper, die von den zwei kongruenten Folien (20), die spiegelbildlich zueinander geschweißt sind, gebildet werden. Die Taschen (31) und (32) bildenden Hohlräume werden durch Bereiche anliegender Flächen (30), die den Spritzenkörper umsäumen, getrennt. Daran schließen sich jeweils die zur Aufnahme der Kanülen länglich geformten Taschen (32) an.

Die als Sollbruchstellen gestalteten Perforationen (40) verlaufen entlang der Ränder des Spritzenkörpers, soweit diese Ränder jeweils innenliegend sind und ermöglichen das Heraustrennen der jeweiligen Injektionsvorrichtung.

Fig. 2 zeigt eine dreidimensionale Darstellung der Erfindung mit Drauf- und Seitenansicht. Die kongruenten, spiegelbildlich zueinander geschweißten Folien zeigt (20). Der zum Heraustrennen weiterhin vorhandene, vorzugsweise verstärkt ausgebildete Rand wird durch (50) dargestellt.

Die Injektionsvorrichtungen als Teile der Multiblockblisterpackung bestehen aus Kunststoffmaterial, vorzugsweise aus Polyethylen, Polypropylen, Polyvinylchlorid oder Polystyrol.

Die Injektionsvorrichtung eignet sich in besonderer Weise, ohne darauf beschränkt zu sein, für Anwendungen von "small volume parenterals". Insbesondere zur intramuskulären oder subcutanen Selbstmedikation ist sie geeignet. Aber auch zur intravenösen Verabreichung von Medikamenten ist sie vorgesehen.

Als Wirkstoffe kommen alle derartige in Frage, die zur Verabreichung mittels Injektionen geeignet sind.

Das Aufnahmevolumen eines durch die erfindungsgemäß ausgeführte Multiblockblisterpackung gebildeten Spritzenkörpers reicht von 0,1 ml bis 30 ml, vorzugsweise 0,1 ml - 1 ml.

Die feste Verbindung des Spritzenkörpers mit der Kanüle kann durch Kleben, Schweißen, Crimpen oder Schrumpfen erfolgen.

Zur Herstellung der erfindungsgemäßen Injektionsvorrichtung, insbesondere des Spritzenkörpers, kann grundsätzlich die Blasformtechnik, auch als "Blow-Fill-Seal-Technology" (BFS-Technology) bekannt, verwendet werden.

Diese Blasform-/Abfüll-/Verschlußtechnik zeichnet sich als zur Zeit geeignete aseptische Verpackungsmethode aus, mit der die jeweils gewünschte Form zuerst geblasen, dann mit dem sterilen Inhalt gefüllt und anschließend in einem Vorgang verschlossen wird.

Die wesentlichen Verfahrensschritte dieser Technologie werden in den Firmenschriften der Unternehmen Waverley Pharmaceutical, Cheshire WA7 1QE, England und Automatic Liquid Packaging, Inc. (ALP), Woodstock, Illinois 60098, USA, beschrieben.

## Patentansprüche

1. Multiblockblisterpackung aus zwei kongruenten, spiegelbildlich gegeneinander geschweißten Folien (20) mit zu Taschen (31,32) geformten Hohlräumen, die mindestens zwei Injektionsvorrichtungen aufweist, von denen jede innerhalb der Multiblockblisterpackung eine selbständige einzelne Grundeinheit von mehreren darstellt und jede einzelne Grundeinheit der Multiblockblisterpackung Bestandteil der besagten zwei kongruenten, spiegelbildlich gegeneinander geschweißten Folien (20) ist, die anliegende (30) und nicht anliegende Bereiche (31) aufweisen, die mit ihren nicht anliegenden Bereichen Taschen (31, 32) bilden, von denen die erste Tasche (31) einen mit Wirkstoff beladenen Spritzenkörper ausmacht, mit dem eine Kanüle fest verbunden ist, und die zweite, länglich geformte Tasche (32) zur schützenden Aufnahme des restlichen Kanülenteils ausgebildet ist, **dadurch gekennzeichnet, daß** die erste Tasche (31) in länglicher Form gestaltet ist und auf ihrer Mittelachse ein konisch zulaufendes Ende aufweist, in das die Kanüle hineinragt, die zweite Tasche (32) in Verlängerung des konisch zulaufenden Endes der ersten Tasche (31) angeordnet ist, und entlang der innenliegenden Schweißränder der den Spritzenkörper ausmachenden ersten Tasche (31) Perforationen (40) vorhanden sind, die als Sollbruchstellen ein Abtrennen eines durch die erste Tasche (31) gebildeten Spritzenkörpers zusammen mit der fest verbundenen Kanüle zum sofortigen Gebrauch aus der Multiblockblisterpackung ermöglichen.

2. Multiblockblisterpackung nach Anspruch 1, **dadurch gekennzeichnet, daß** an der Seite der Multiblockblisterpackung, zu der die Kanülen hinweisen, ein durch eine Schweißnaht gebildeter Rand (50) von mindestens 10 mm Breite über die Gesamtlänge der Multiblockblisterpackung vorhanden ist, der verstärkt sein kann.

3. Multiblockblisterpackung nach einem der Ansprüche 1 oder 2, **dadurch gekennzeichnet, daß** sie fünf bis zehn Injektionsvorrichtungen aufweist.

## Claims

1. Multiblock blister pack formed from two congruent films (20) welded to one another in mirror image fashion with hollow spaces formed into pockets (31, 32), having at least two injection devices of which each represents one independent basic unit out of a plurality within the multiblock blister pack and each individual basic unit of the multiblock blister pack is a component of the two congruent films (20) having contacting (30) and non-contacting (31) regions which with their non-contacting regions form pockets (31, 32) of which the first pocket (31) comprises a syringe body charged with medication, to which a cannula is firmly attached, and the second elongated pocket (32) is formed for protective accommodation of the remaining part of the cannula, **characterised in that** the first pocket (31) is made in elongated form and has an end which narrows conically about its mid-axis and into which the cannula projects, the second pocket (32) is arranged as extension of the conically narrowing end of the first pocket (31), and along the interior weld edges of the first pocket (31) comprising the syringe body perforations (40) are present, which, as intended break sites, enable separation out of the multiblock blister pack of a syringe body formed by the first pocket (31) together with the firmly attached cannula, for immediate use.

2. Multiblock blister pack according to Claim 1, **characterised in that** on the side of the multiblock blister pack towards which the cannulae point, an edge (50), which may be reinforced, of at least 10 mm width formed by a weld seam is present along the entire length of the multiblock blister pack.

3. Multiblock blister pack according to one of the claims 1 or 2, **characterised in that** it has between five and ten injection devices.

## Revendications

1. Emballage blister multibloc composé de deux feuilles (20) congruentes soudées l'une à l'autre en symétrie spéculaire, avec des cavités formées en poches (31, 32) qui présentent au moins deux dispositifs d'injection, dont chacun représente, au sein de l'emballage blister multibloc, une unité de base autonome parmi plusieurs et chaque unité de base de l'emballage blister multibloc fait partie desdites deux feuilles (20) congruentes soudées l'une à l'autre en symétrie spéculaire, qui présentent des zones adjacentes (30) et des zones non adjacentes (31), qui forment avec leurs zones non adjacentes des poches (31, 32) dont la première poche (31) constitue un corps de seringue chargé en substance active et auquel est reliée solidement une aiguille, et la deuxième poche (32) de forme allongée est formée pour recevoir de manière protectrice la partie restante de l'aiguille, ***caractérisé en ce que*** la première poche (31) est réalisée de forme allongée et présente sur son axe médian une extrémité effilée conique sur laquelle l'aiguille fait saillie, la deuxième poche (32) est placée dans le prolongement de l'extrémité effilée conique de la première poche (31), et le long des bords soudés intérieurs de la première poche (31) constituant le corps de seringue sont placées des perforations (40) qui, en tant que zones de rupture imposée, permettent une séparation d'avec l'emballage blister multibloc, d'un corps de seringue formé par la première poche (31) avec l'aiguille solidement reliée en vue d'une utilisation immédiate.

2. Emballage blister multibloc selon la Revendication 1, ***caractérisé en ce que,*** sur le côté de l'emballage blister multibloc vers lequel sont orientées les aiguilles est présent un bord (50) formé d'un joint soudé d'au moins 10 mm de large sur toute la longueur de l'emballage blister multibloc, bord qui peut être renforcé.

3. Emballage blister multibloc selon l'une quelconque des Revendications 1 ou *2,* ***caractérisé en ce qu***'il présente de cinq à dix dispositifs d'injection.
